# EUROPEAN PATENT SPECIFICATION

(11) **EP 2 629 664 B1**
(45) Date of publication and mention of the grant of the patent: **30.12.2015**
(21) Application number: 11778704.4
(22) Date of filing: 12.10.2011
(51) Int. Cl.: A61B 5/16, G06F 19/00

(54) **ANXIETY MONITORING**
ÜBERWACHUNG VON ANGSTGEFÜHL
SURVEILLANCE DE L'ANXIÉTÉ

(30) Priority: 19.10.2010 EP 10188038
(43) Date of publication of application: 28.08.2013
(73) Proprietor: Koninklijke Philips N.V., 5656 AE Eindhoven (NL)
(72) Inventor: VAN ELSWIJK, Gijs, Antonius, Franciscus, NL-5656 AE Eindhoven (NL); DESSAUD, Nathalie, Magali, Danielle, NL-5656 AE Eindhoven (NL); TIJS, Tim, Johannes, Willem, NL-5656 AE Eindhoven (NL); GILLIES, Murray, Fulton, NL-5656 AE Eindhoven (NL); VOGT, Jürgen, NL-5656 AE Eindhoven (NL)
(74) Representative: Steffen, Thomas
(86) International application number: PCT/IB2011/054496
(87) International publication number: WO 2012/052880

(56) References cited:
- WO-A1-02/085198
- WO-A1-2008/064431
- WO-A2-2007/123799
- WO-A2-2008/055078
- WO-A2-2008/099288
- WO-A2-2011/109716
- US-A1- 2003 139 946
- US-A1- 2006 116 555

## Description

### FIELD OF THE INVENTION

The invention pertains to a patient monitoring system having the function to assess stress or anxiety with a patient to be examined.

### BACKGROUND OF THE INVENTION

Such a patient monitoring system is known from the paper 'Clinical decision - support for diagnosis stress-related disorders by applying psychophsiological medical knowledge to an instance -based learning system' by M. Nilsson et al. in Artificial Intelligence in Medicine 36(2006)159-176. This known patient monitoring system classifies heart-rate patterns. The classified heart-rate patterns indicate if a patient may be suffering from a stress-related disorder.

US 2006/0116555 A1 discloses a controlled environment thermal image detection system. This system may be used for determining a physiological state of a person.

A further system for monitoring emotional state changes is known from WO 2008/064431 A1.

WO 2008/055078 A2 discloses a method for automatically identifying emotional states of a person in real-time based on physiological responses by exposing a person to a stimulus, measuring the person's physiological response to the stimulus, and comparing the measured physiological response to a known baseline physiological response.

WO 02/085198 A1 discloses a system including thermal image analysis for polygraph testing. Therein, thermal image data of at least a region of a face of a person is provided. The thermal image data is transformed to blood flow rate data which includes a slope representative of the change of blood flow rate over time. This slope is compared to a slope threshold to determine a physiological state of the person.

WO 2008/099288 A2 discloses a portable, handheld biosensor device that is held between two fingers of the same hand. The sensor device includes a pair of conductive or semi-conductive electrodes for measuring the electrical conductance of the skin. The measured biometric values are transmitted wirelessly to a computing device where the data is utilized to generate a control parameter in a software application whose purpose is to provide anxiety biofeedback or entertainment.

A further method of collecting data on anxiety disorders is known from US 2003/0139946 A1.

WO 2007/123799 A2 discloses a method of assessing subject's reactivity to psychological stress using quantitative functional MRI.

### SUMMARY OF THE INVENTION

An object of the present invention is to efficiently and effectively put a patient to be examined at ease. Notably, the present invention addresses to reduce patient's level of anxiety when the patient needs to undergo an examination in an imaging modality such as an x-ray examination system, a magnetic resonance examination system, a PET system, a computed tomography system or a hybrid imaging system such as a PET/MRI system or a PET/CT system.

This object is achieved by a patient monitoring system according to claim 1 comprising:
- a probe module to acquire data of a patient to be monitored
- an evaluation module to determine a patient's state of anxiety on the basis of the acquired data and
- an indicator module to feedback the determined state of anxiety to attending staff. The probe module includes a salivary cortisol assay to measure a salivary cortisol level. The evaluation moduel is configured to determine the patient's state on the basis of the salivary cortisol level.

On the basis of the determined state of patient anxiety, the attending staff is informed. Notably, the state of anxiety includes the level of anxiety of the patient in a quantitative measure. Consequently, the staff can efficiently and effectively approach the more anxious patients in a re-assuring way to put those patients at ease. Thus, the staff is enabled on the basis of the feedback from the patient monitoring system to re-assure patients where that is needed and beneficiary. Notably, the invention avoids that staff approaches patients that are not so anxious and incur risks that those patients are rendered more anxious because of unnecessary staff attention. Further the invention reduces exposure of hospital staff to radiation and thus avoids that hospital staff is unnecessarily exposed to radiation (in for example nuclear medicine (PET) examinations).

The invention enables a more reliable assessment of the patient's anxiety level because the assessment is not affected by personal interaction between member(s) of staff and the patient. The staff's attention is accurately customised to the individual patient because the feedback to the staff is based on the data acquired from the patient at issue. In this way, extra time spent by staff on an objectively more anxious patient will lead to a benefit in less time required for the patient who is more susceptible to anxiety during the actual medical procedure.

Examples of such medical procedure are imaging examinations e.g. by magnetic resonance imaging or positron emission tomography that requires a radioactive tracer. These are expensive imaging procedures for which it is to be avoided that they would need to be re-iterated because of restlessness of the anxious patient. Another benefit of the patient monitoring system of the invention is that it can help minimize exposure time of hospital staff to radiation in medical procedures that involve radioactive tracers. Finally, this invention provides a more personalized patient care which contributes to overall improvement of patient well-being.

The invention takes account of a wide variety of anxiety levels of different patients and different types of medical procedures. In particular, individual patients may react differently on various types of medical procedures, like an imaging examination procedure based on x-ray imaging, computed tomography or magnetic resonance imaging.

Further, the invention facilitates formal implementation of an anxiety management protocol in a healthcare institution, such as in a hospital or point of care, because it allows different anxiety management strategies to different anxiety profiles, coping styles, and anxiety levels. The invention enables to generate decisions to be better customised to the patient's needs and to give a patient extra attention independent form a individual subjective anxiety assessment of the physician or nurse involved at any particular time. Thus the way a patient is approached or guided can vary extensively.

The recommendations or assessments of this invention create more consistency in patient-staff contact in a given department. Once a patient profile has been determined it can be stored to help improve efficiency in planning of return visits, for example by avoiding planning of anxious patients at a concurrent timeslot. The feedback to the staff may be provided in different forms. For example the patient monitoring system includes a user interface with a display on which the patient's level of anxiety can be displayed. An attractive way of displaying the patient's level of anxiety is as a 'traffic light'. For procedures where multiple patients are monitored or the nurse is mobile then a portable display device (e.g. "iPhone"or "iPad") can be the preferred interface.

These and other aspects of the invention will be further elaborated with reference to the embodiments defined in the dependent Claims.

In one aspect of the patient monitoring system of the invention, the probe module is configured to acquire primary data which are employed by the evaluation module to generate a primary assessment of the patient's level of anxiety. Preferably, also a reliability score for the assessed level of anxiety is calculated. The reliability score can be a statistical spread measure such as the standard deviation, or coefficient of variation.

Instead of the above simple statistical spread measure, more sophisticated algorithms might be used to combine information from multiple sources to determine the probability and/or reliability scores, such as conditional or Bayesian probabilities, decision trees, etc.

When on the basis of the primary assessment of the patient's anxiety level, optionally also taking the calculated confidence range into account, additional data may be acquired and employed to make a more refined assessment of the patient's anxiety level. In particular, in this aspect of the invention, the primary data acquisition is less obtrusive for the patient and/or the staff. Thus, it is achieved that more obtrusive additional data acquisition is activated only when there is a real need to do so. In other words, more obtrusive data acquisition is avoided when the patient's anxiety level could be reliably assessed on the basis of the primary data, which only requires a less obtrusive approach of the patient.

In a further aspect of the invention the patient monitoring system of the invention is provided with a self-learning function. In this aspect the patient monitoring system is provided with a user interface via which the staff can confirm or override the evaluated anxiety level. This input from the staff is stored and used to update the relationship between the acquired data and the to be evaluated anxiety level. Then the updated relationship is stored and made available to the evaluation module for future use. This self-learning aspect may be assigned to an individual patient but can also be used in the evaluation of anxiety of any patient from a wider group. For example, over time it might become evident that anxiety levels for lymphoma patients on their first PET-scan is typically much higher than in follow-up scans.

In another aspect of the invention, the evaluation module is provided with, or provided with an access to, stored patient reference data that are related to pre-determined anxiety levels. Multiple parameters in the reference dataset that are related to the pre-determined anxiety level yield more reliable estimates of the anxiety level of the patient. Examples of reference data are demographic profiles that include patient's gender, age, smoking behaviour, education level, or personality traits such as trait-anxiety or preferred coping style.

In another aspect of the invention the probe module is provided with a camera system that acquires image information of the patient. Notably, the camera system can be configured to image several patients simultaneously, e.g. while they are in the waiting room before undergoing the medical procedure. From the image information several parameters of the imaged patient can be derived, such as heart rate, respiration rate, motion, facial expression, pupil size (diameter). Image based respiration monitoring as such is mentioned in the European patent application 10160571.5 (PH014905). By comparing one or more of these parameters to reference distributions the anxiety level of the patient can be assessed

In a further aspect of the invention, the probe module is provided with a physiology sensor. The evaluation module evaluates the patient's level of anxiety from the physiological data acquired by the physiology sensor. For example, the physiology sensor may be a temperature sensor to measure the patient's skin temperature, or a conductivity sensor (Ohm meter) to measure the electrical conductivity of the patient's skin.

In a next aspect of the invention, the probe module is configured to present the patient with a questionnaire. Example of a typical questionnaire is the Spielberger's State Anxiety Inventory that consist of 20 questions, such as: "I feel calm", "I feel secure", "I am tense". Each of the questions has to be responded to on a discrete 5-point scale (called a Likert scale) from 1 (not at all) to 5 (very much so).

The evaluation module derives the level of anxiety from the responses to the questionnaire. For most psychological questionnaires norm tables are published for several reference populations (e.g. males, elderly, students, etc).

In the invention, the probe module includes a cortisol assay which measures the cortisol in the patient's saliva. Alternatively, blood cortisol level may be employed. It is known per se that psychological stress, leads to the release of corticotropin releasing hormone and adrenocorticotropic hormone with a subsequent rise in cortisol levels Kirschbaum, C., & Hellhammer, D. (1994). 'Salivary cortisol in psychoneuroendocrine research:Recent developments and applications.' in Psychoneuroendocrinology, 19(1994)313-333.

In a further aspect of the invention, the evaluation module is further configured to take availability of staff into account for providing feedback on the patient's state of anxiety to the staff. In this implementation the evaluation module has access to e.g. staff work schedule as planned or information of staff's current activities. For example the evaluation module is configured to lower feedback if there is less staff available and provide feedback limited to patient's showing higher levels of anxiety. In this way the patient monitoring system of the invention improves the efficiency of the deployment of staff. Notably, when staff is more busy, they are less provided with feedback on lesser anxious patient's because there will not be staff available to attend and re-assure those lesser anxious patients. On the other hand, the more staff is available, the evaluation module controls the indicator module to also provide feedback on the lesser anxious patients.

In another aspect, the invention is applied in the field of nuclear medicine, in particular in conjunction with PET (positron emission tomography. In this embodiment the probe module has the further function to measure radioactivity of a radioactive tracer contrast agent prepared to be administered to the patient to be examined. The radioactivity can be measured with a semiconductor radiation sensor or with a Geiger counter type of device. The measured level of radioactivity is applied to the evaluation module. The evaluation module is configured to determine an optimum instant for administering, e.g. by injection or infusion, the radioactive tracer contrast agent and the patient's state of anxiety. An object of application of the invention in nuclear medicine is to improve clinical workflow. It is observed that in nuclear medicine conventional practices to reduce stress, such as massage therapy, are not conventionally applied because of the risk to deteriorate the uptake of radioactive contrast agent. In nuclear medicine, the workflow is dominated by the requirements due to the half-life of the radioactive tracer contrast agent. For example radioactive ¹⁸F has a rather short half-life of about 110minutes and needs to be administered to the patient e.g. in the form of fluor-deoxyglucose (¹⁸FDG) rather short before the actual PET scan is performed. The present invention enables to apply the radioactive contrast agent optimally when the patient is relatively relaxed while the radioactivity is relatively high. Because patient anxiety (or stress) level is lower, perturbations due to patient stress on the acquired image data are avoided and the diagnostic image quality is improved. In particular, as the patient's anxiety level is lower, tumour-background radioactivity in the image data is higher and the level of false positives in the image is lower. Notably, as the anxiety level of the patient to be examined is lower, abnormal hypermetabolistic areas that may not contain cancer cells, but are e.g. due to muscular activity is avoided. On the other hand, when the instant for administering the radioactive contrast agent is optimised, excessive exposure to radioactivity can be avoided without negatively affecting diagnostic image quality. Accordingly, the invention enables to optimise the instant of administering the radioactive contrast agent, reduce the anxiety level of the patient during uptake of the contrast agent as well as during the actual acquisition of image data, e.g. during the PET scan. These effects are moreover achieved together with in improved clinical workflow in which attending staff is more efficiently deployed and less exposed to radioactivity. Workflow is even further improved by taking staff availability into account together with the measured radioactivity. In a practical example of the invention, the probe module is formed as a bracelet to be worn by the patient. The bracelet or wristband incorporates both a sensor to acquire the data of the patient from which the state of anxiety is determined as well as a radioactivity sensor. For example, a semiconductor radiation sensor and a senor for measuring electrical skin conductivity can be incorporated in such a bracelet. The bracelet is non-obtrusive for the patient and does not inherently add to the patient's anxiety.

These and other aspects of the invention will be elucidated with reference to the embodiments described hereinafter and with reference to the accompanying drawing wherein

### BRIEF DESCRIPTION OF THE DRAWINGS

Fig. 1 shows a diagrammatic representation of a patient monitoring as implemented at a point-of-care and
in Fig. 2 another example of invention applied in nuclear medicine is schematically depicted.

### DETAILED DESCRIPTION OF THE EMBODIMENTS

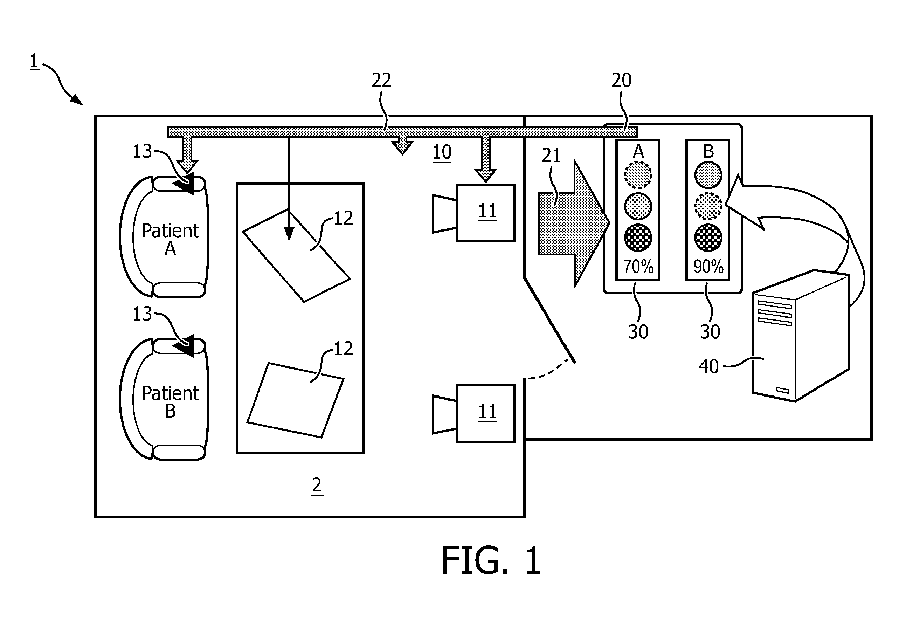

Figure 1 shows a diagrammatic representation of a patient monitoring as implemented at a point-of-care, such as a physician's office or an imaging modality suite. The patient monitoring system 1 of the invention comprises the probe module 10, which acquires data from the patient to be examined. In the example of Figure 1 two camera's 11 are provided to image the motion due to heart rate, respiratory pattern, motion, pupil size (diameter) and the facial expression of the patient. From an image analysis of these images, the anxiety level of the patient is assessed. To that end the evaluation module is provided with an image analysis and an assessment function that is usually implemented in software. In the set up shown in Figure 1, two patients, patient A and patient B, in the waiting room 2, can be monitored individually. For each of the patient's a photoplethysmography sensor 13 is provided to acquire the patient's pulse rate. Further, questionnaire consoles 12 are provided which dispense access to questionnaires for each of the patients. For example state anxiety questionnaires or trait anxiety questionnaire may be employed. The questionnaire consoles output the patient's responses to these questionnaires. In secondary stages a "trait" questionnaire is used that will provide information about relevant personality characteristics such as coping style, or trait anxiety. Examples would be: Hospital Anxiety and Depression Scale; Spielberger's Trait Anxiety Inventory and Coping Operations Preference Enquiry.

The data acquired by the probe module 10 are applied to the evaluation module 20 over a data connection 21. On the basis of the data from the probe module, the evaluation module computes anxiety estimates for the individual patients. The evaluation module supplies its output anxiety estimate to the indicator module 30, for example in the form a 'traffic lights' for each individual patient. Thus the evaluation module assesses the patient's anxiety level and feedbacks the assessment to the staff. The 'traffic lights' may be further provided with a feedback option, e.g. in the form of a button that can be applied by the staff in response to the indication generated by the evaluation module 20. The staff's feedback may be to confirm or to overrule the evaluation module's assessment. The staff's feedback is used by the evaluation module's self-learning function to improve its accuracy.

Further, the evaluation module 20 is provided with access to stored reference data in a memory unit 40. The memory unit contains demographic profiles, including the patient's gender, age, type of examination, total number of visits.

The evaluation module also has the function to assess the patient's anxiety level at several hierarchical levels and control the probe module to perform additional data in conformity with the hierarchy level of the assessment. The hierarchical levels involve for example a primary, and further more increasingly refined assessments that require primary and additional data, respectively, to the acquired. For example, already in the primary assessment a reliability score of the assessed anxiety level is calculated on the basis of which the next hierarchical level is activated. In an example of this: Patient is an adult male. The first variable is the heart rate measured at 120 beats/minute. The evaluation system knows the probability distribution function of the heart rate for stressed males, and thereby determines that the probability that a male under stress has a heart rate ≤ 120 is 90%. In a similar fashion, the stress probability is computed for a second and third variable (e.g. respiration rate and pupil size). For these variables the respective stress probabilities are 40% and 80%. This leads to the average stress probability of 70% (i.e. a red traffic light), with the standard deviation of 26% as a reliability index.

Increasing hierarchy has an increasing level of obtrusiveness for the patient to be examined. To control raising to the next level, the patient monitoring system comprises a control bus over which control signals are transferred from the evaluation module 20 to the probe module 10, notably to the data acquisition components of the probe module, such as the camera's, questionnaire consoles etc. The evaluation module is configured to turn to a higher hierarchical level if the assessment at the current level has a confidence level below pre-set threshold.

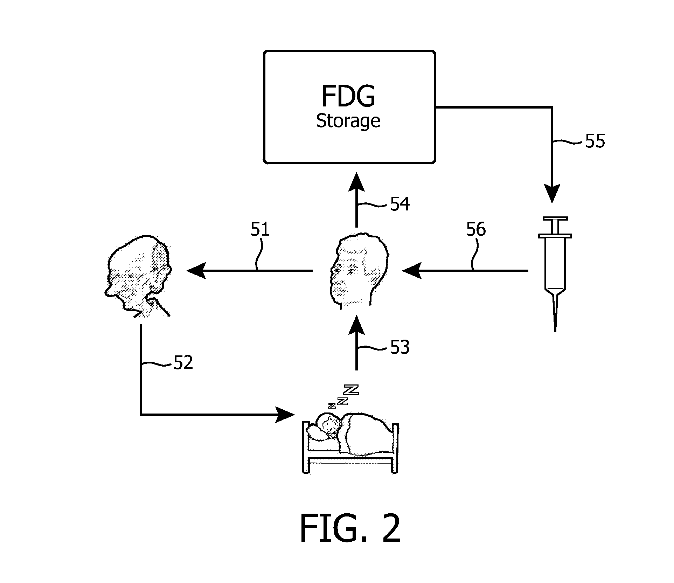

Another example of invention applied in nuclear medicine is schematically depicted in Figure 2: Patient stress level is communicated at an early stage to the clinician 51, to help the clinician take the appropriate steps (e.g., schedule extra time if expected necessary) for patient relaxation. Next, the clinician aims to relax the patient, possibly assisted by technology 52 > 53. As soon as the injection system notices the patient is sufficiently relaxed, the FDG storage unit is triggered 54. The FDG storage unit determines the optimal FDG quantity (given the desired dose and current radioactivity) and sends the FDG to the injection unit 55, which injects the FDG into the patient 56.

## Claims

1. A patient monitoring system comprising:
- a probe module (10) to acquire data of a patient to be monitored,
- an evaluation module (20) to determine a patient's state of anxiety on the basis of the acquired data, and
- an indicator module (30) to feedback the determined state of anxiety to attending staff,
**characterized in that** the probe module (10) includes a salivary cortisol assay to measure a salivary cortisol level, and **in that** the evaluation module (20) is configured to determine the patient's state on the basis of the salivary cortisol level.

2. The patient monitoring system as claimed in Claim 1, wherein
- the probe module (10) is configured to acquire primary data and optionally to acquire additional data of the patient
- the evaluation module (20) is configured
- to make a primary assessment of the patient's state of anxiety from the primary data and optionally make a refined assessment of the patient's state of anxiety on the basis of the additional data and
- to control the probe module (10) on the basis of the primary assessment to acquire the additional data and to engage in the refined assessment.

3. The patient monitoring system as claimed in Claim 1, wherein the evaluation module (20) includes a self-learning function.

4. The patient monitoring system as claimed in Claim 1, wherein the evaluation module (20) is configured to access patient reference data and to evaluate the patient's state taking the patient reference data into account.

5. The patient monitoring system as claimed in Claim 1, wherein the probe module (10) includes a camera to monitor the patient and an image-evaluation module to evaluate the patient's state of anxiety on the basis of image information acquired by the camera.

6. The patient monitoring system as claimed in Claim 1, wherein the probe module (10) includes a physiology sensor and an physiology-evaluation module to evaluate the patient state of anxiety on the basis of physiological data acquired by the physiology sensor.

7. The patient monitoring system as claimed in Claim 1, wherein the probe module (10) is arranged to present a questionnaire to the patient and receive responses to the questionnaire.

8. The patient monitoring system as claimed in Claim 1, wherein
the evaluation module (20) is further configured to take staff availability into account for controlling the indicator module to feedback the state of anxiety.

9. The patient monitoring system as claimed in Claim 1, wherein
- the probe module (10) also has the function to measure radioactivity of a radioactive tracer prepared to be administered to the patient and
- the evaluation module (20) is configured to
- receive the measured radioactivity level,
- to determine an optimum instant for administering the radioactive tracer to the patient on the basis of the measured radioactivity level and the state of anxiety of the patient.

10. The patient monitoring system as claimed in Claim 1, wherein
- the probe module (10) has the function to measure radioactivity of a radioactive tracer that has been administered to the patient and
- the evaluation unit (20) is configured to control the indicator module (30) to feedback to the attending staff on the basis of the patient's state of anxiety and the measured level of radioactivity.

11. A computerized patient monitoring method including
- acquisition of data of a patient to be monitored,
- determination of an indication of a patient's state of anxiety on the basis of the acquired data, and
- feedback the determined indication of the state of anxiety to attending staff,
**characterized in that** the acquisition of data includes a cortisol assay which measures a cortisol level in the patient's saliva, and **in that** the patient's state is determined on the basis of the salivary cortisol level.

12. A computer programme including instructions to
- acquire data of a patient to be monitored,
- determine a patient's state of anxiety on the basis of the acquired data, and
- feedback the determined state of anxiety to attending staff,
**characterized in that** the acquisition of data includes a cortisol assay which measures a cortisol level in the patient's saliva, and **in that** the patient's state is determined on the basis of the salivary cortisol level.

## Patentansprüche

1. Patientenüberwachungssystem, das Folgendes umfasst:
- ein Fühlermodul (10) zum Erfassen von Daten eines zu überwachenden Patienten,
- ein Bewertungsmodul (20) zum Ermitteln eines Angstzustands des Patienten auf der Grundlage der erfassten Daten, und
- ein Anzeigemodul (30) zum Rückmelden des ermittelten Angstzustands an das betreuende Personal,
**dadurch gekennzeichnet, dass** das Fühlermodul (10) ein Speichel-Cortisol-Assay zum Messen eines Speichel-Cortisol-Spiegels umfasst, und dass das Bewertungsmodul (20) dafür eingerichtet ist, den Zustand des Patienten anhand des Speichel-Cortisol-Spiegels zu ermitteln.

2. Patientenüberwachungssystem nach Anspruch 1, wobei
- das Fühlermodul (10) dafür eingerichtet ist, Primärdaten zu erfassen und wahlweise zusätzliche Daten des Patienten zu erfassen,
- das Bewertungsmodul (20) dafür eingerichtet ist,
- eine Primäreinschätzung des Angstzustands des Patienten anhand der Primärdaten vorzunehmen und eine verfeinerte Einschätzung des Angstzustands des Patienten auf der Grundlage der zusätzlichen Daten vorzunehmen und
- das Fühlermodul (10) auf der Grundlage der Primäreinschätzung zu steuern, um die zusätzlichen Daten zu erfassen und die verfeinerte Einschätzung einzuleiten.

3. Patientenüberwachungssystem nach Anspruch 1, wobei das Bewertungsmodul (20) eine Selbstlernfunktion umfasst.

4. Patientenüberwachungssystem nach Anspruch 1, wobei das Bewertungsmodul (20) dafür eingerichtet ist, auf Patientenreferenzdaten zuzugreifen und den Zustand des Patienten unter Berücksichtigung der Patientenreferenzdaten zu bewerten.

5. Patientenüberwachungssystem nach Anspruch 1, wobei das Fühlermodul (10) eine Kamera zum Überwachen des Patienten und ein Bildbewertungsmodul zum Bewerten des Angstzustands des Patienten anhand der durch die Kamera erfassten Bildinformationen umfasst.

6. Patientenüberwachungssystem nach Anspruch 1, wobei das Fühlermodul (10) einen Physiologie-Sensor und ein Physiologie-Bewertungsmodul umfasst, um den Angstzustand des Patienten anhand der durch den Physiologie-Sensor erfassten physiologischen Daten zu bewerten.

7. Patientenüberwachungssystem nach Anspruch 1, wobei das Fühlermodul (10) dafür ausgelegt ist, dem Patienten einen Fragebogen vorzulegen und Antworten auf den Fragebogen zu empfangen.

8. Patientenüberwachungssystem nach Anspruch 1, wobei
das Bewertungsmodul (20) weiterhin dafür eingerichtet ist, die Verfügbarkeit des Personals zum Kontrollieren des Anzeigemoduls zum Rückmelden des Angstzustands zu berücksichtigen.

9. Patientenüberwachungssystem nach Anspruch 1, wobei
- das Fühlermodul (10) auch die Funktion hat, die Radioaktivität eines radioaktiven Tracers zu messen, der für die Verabreichung an den Patienten vorbereitet ist, und
- das Bewertungsmodul (20) dafür eingerichtet ist,
- den Messwert für den Radioaktivitätspegel zu empfangen,
- einen optimalen Zeitpunkt zur Verabreichung des radioaktiven Tracers an den Patienten auf der Grundlage des gemessenen Radioaktivitätspegels und des Angstzustands des Patienten zu ermitteln.

10. Patientenüberwachungssystem nach Anspruch 1, wobei
- das Fühlermodul (10) die Funktion hat, die Radioaktivität eines radioaktiven Tracers zu messen, der dem Patienten verabreicht worden ist, und
- die Bewertungseinheit (20) dafür eingerichtet ist, das Anzeigemodul (30) zu steuern, um dem betreuenden Personal Rückmeldungen auf der Grundlage des Angstzustands und des gemessenen Radioaktivitätspegels zu liefern.

11. Computerisiertes Patientenüberwachungsverfahren, das Folgendes umfasst:
- Erfassen von Daten eines zu überwachenden Patienten,
- Ermitteln einer Angabe eines Angstzustands des Patienten auf der Grundlage der erfassten Daten, und
- Rückmelden der ermittelten Angabe des Angstzustands an das betreuende Personal,
**dadurch gekennzeichnet, dass** die Erfassung der Daten ein Cortisol-Assay umfasst, das einen Cortisol-Spiegel in dem Speichel des Patienten misst, und dass der Zustand des Patienten anhand des Speichel-Cortisol-Spiegels ermittelt wird.

12. Computerprogramm mit Anweisungen zum
- Erfassen von Daten eines zu überwachenden Patienten,
- Ermitteln eines Angstzustands des Patienten auf der Grundlage der erfassten Daten, und
- Rückmelden des ermittelten Angstzustands an das betreuende Personal,
**dadurch gekennzeichnet, dass** die Erfassung der Daten ein Cortisol-Assay umfasst, das einen Cortisol-Spiegel in dem Speichel des Patienten misst, und dass der Zustand des Patienten anhand des Speichel-Cortisol-Spiegels ermittelt wird.

## Revendications

1. Système de surveillance de patient comprenant :
- un module de sonde (10) pour acquérir des données d'un patient à surveiller,
- un module d'évaluation (20) pour déterminer l'état d'anxiété d'un patient sur la base des données recueillies, et
- un module indicateur (30) pour rapporter l'état d'anxiété déterminé à un personnel présent,
**caractérisé en ce que** le module de sonde (10) comprend un dosage de cortisol salivaire pour mesurer un taux de cortisol salivaire, et **en ce que** le module d'évaluation (20) est conçu pour déterminer l'état du patient sur la base du taux de cortisol salivaire.

2. Système de surveillance de patient selon la revendication 1, dans lequel
- le module de sonde (10) est conçu pour acquérir des données primaires et éventuellement pour acquérir des données supplémentaires du patient
- le module d'évaluation (20) est conçu
- pour faire une évaluation primaire de l'état d'anxiété du patient à partir des données primaires et faire éventuellement une évaluation plus poussée de l'état d'anxiété du patient sur la base des données supplémentaires et
- pour commander le module de sonde (10) sur la base de l'évaluation primaire pour acquérir les données supplémentaires et pour s'engager dans l'évaluation plus poussée.

3. Système de surveillance de patient selon la revendication 1, dans lequel le module d'évaluation (20) comprend une fonction d'autoapprentissage.

4. Système de surveillance de patient selon la revendication 1, dans lequel le module d'évaluation (20) est conçu pour accéder à des données de référence du patient et pour évaluer l'état du patient en tenant compte des données de référence du patient.

5. Système de surveillance de patient selon la revendication 1, dans lequel le module de sonde (10) comprend une caméra pour surveiller le patient et un module d'évaluation d'image pour évaluer l'état d'anxiété du patient sur la base des informations d'image recueillies par la caméra.

6. Système de surveillance de patient selon la revendication 1, dans lequel le module de sonde (10) comprend un capteur de physiologie et un module d'évaluation de physiologie pour évaluer l'état d'anxiété du patient sur la base de données physiologiques recueillies par le capteur de physiologie.

7. Système de surveillance de patient selon la revendication 1, dans lequel le module de sonde (10) est disposé pour présenter un questionnaire au patient et recevoir des réponses au questionnaire.

8. Système de surveillance de patient selon la revendication 1, dans lequel
le module d'évaluation (20) est en outre conçu pour tenir compte de la disponibilité du personnel pour commander le module indicateur pour rapporter l'état d'anxiété.

9. Système de surveillance de patient selon la revendication 1, dans lequel
- le module de sonde (10) possède également la fonction de mesure de la radioactivité d'un traceur radioactif préparé pour être administré au patient et
- le module d'évaluation (20) est conçu
- pour recevoir le niveau mesuré de radioactivité,
- pour déterminer un moment optimal pour administrer le traceur radioactif au patient sur la base du niveau mesuré de radioactivité et de l'état d'anxiété du patient.

10. Système de surveillance de patient selon la revendication 1, dans lequel
- le module de sonde (10) possède la fonction de mesure de la radioactivité d'un traceur radioactif qui a été administré au patient et
- l'unité d'évaluation (20) est conçue pour commander le module indicateur (30) pour rapporter au personnel présent sur la base de l'état d'anxiété du patient et du niveau mesuré de radioactivité.

11. Procédé informatisé de surveillance d'un patient, comprenant
- l'acquisition de données d'un patient à surveiller,
- la détermination d'une indication de l'état d'anxiété d'un patient sur la base des données recueillies, et
- le retour de l'indication déterminée de l'état d'anxiété au personnel présent,
**caractérisé en ce que** l'acquisition de données comprend un dosage de cortisol qui mesure un taux de cortisol dans la salive du patient, et **en ce que** l'état du patient est déterminé sur la base du taux de cortisol salivaire.

12. Programme d'ordinateur comprenant des instructions pour
- l'acquisition de données d'un patient à surveiller,
- la détermination d'un état d'anxiété d'un patient sur la base des données recueillies, et
- le retour de l'état d'anxiété déterminé au personnel présent,
**caractérisé en ce que** l'acquisition de données comprend un dosage de cortisol qui mesure un taux de cortisol dans la salive du patient, et **en ce que** l'état du patient est déterminé sur la base du taux de cortisol salivaire.
